(19) Europäisches Patentamt

European Patent Office

Office européen des brevets

(11) **EP 0 887 060 B1**

(12) **EUROPEAN PATENT SPECIFICATION**

(45) Date of publication and mention
of the grant of the patent:
**26.04.2006   Bulletin 2006/17**

(51) Int Cl.:
***A61F 13/15*** (2006.01)

(21) Application number: **97110380.9**

(22) Date of filing: **25.06.1997**

(54) **Dual absorbent core element system with high fluid handling capability insert core**

Doppelschichtiger absorbierender Einsatzkern mit verbesserter Flüssigkeitshandhabung

Insert de noyau absorbant à deux couches avec des propriétés de traitement des fluides améliorés

(84) Designated Contracting States:
**AT BE CH DE DK ES FI FR GB GR IE IT LI LU NL PT SE**

(43) Date of publication of application:
**30.12.1998   Bulletin 1998/53**

(73) Proprietor: **THE PROCTER & GAMBLE COMPANY Cincinnati, Ohio 45202 (US)**

(72) Inventors:
 • **Klodt, Ute**
 **60437 Frankfurt a.M. (DE)**
 • **Blance, Agustin Ramos**
 **61440 Oberursel (DE)**
 • **Ollivier, Ivan Jean**
 **60316 Frankfurt a.M. (DE)**

(74) Representative: **Canonici, Jean-Jacques et al
Procter & Gamble European Service GmbH,
Sulzbacher Strasse 40-50
65824 Schwalbach am Taunus (DE)**

(56) References cited:
**EP-A- 0 557 047         EP-A- 0 719 531
EP-A- 0 774 242         WO-A-94/16659
WO-A-96/29037         GB-A- 2 144 995
GB-A- 2 197 206         US-A- 4 501 587
US-A- 5 236 428**

## Description

<u>Field of the Invention</u>

The present invention relates to disposable absorbent articles such as diapers, incontinence articles, sanitary towels, training pants and the like, and in particular to such articles comprising two absorbent elements, whereby the outer element comprises the primary fixation means and the inner element comprises a high performance absorbent element.

<u>Background of the Invention</u>

Garments to collect body exudates are well known in the art. These garments are designed to receive and contain urine, faeces and other bodily discharged materials and to isolate these discharges both from the body and from the surroundings of the wearer. Typically, these garments have two main parts, namely one absorbent element and fixation and sealing elements, such as tapes, elastics and the like. In some embodiments, both parts form a unitary absorbent article, such as many baby diapers as presently sold in the market, in other embodiments the two parts are not unitary, such as when combining absorbent pads with elasticised pants. Other conventional approaches use a unitary article comprising both absorbent elements and fixation elements in combination with "inserts" to boost the capacity of the first absorbent element to compensate for its insufficient absorbency performance. WO 96/29037, for example, discloses an insert for an absorbent article suitable to form together with this absorbent article a combined absorbent article of increased capacity.
Overall, all emphasis has been spent against improving the performance of the primary element, aiming at eliminating the inner, secondary absorbency element.
However, all these approaches failed to recognise the benefits which arise, when combining an outer element having at least a moderate absorbency performance with highly performing inner elements.

<u>Objects of the invention</u>

Hence it is an object of the present invention to provide a non-unitary absorbent article comprising an outer absorbent element comprising a primary absorbent element, and comprising an inner element comprising a secondary absorbent element of superior skin dryness performance. It is a further object of the present invention to provide a non-unitary absorbent article, whereby one of its elements is intended to be removed and discarded upon each loading, and the other one can be used for several changes of the first.

<u>Summary</u>

The present invention relates to absorbent articles which comprise at least two elements, whereby the element positioned towards the wearer has a higher ultimate storage capacity than the outer one, providing a superior skin dryness performance.

<u>Brief description of drawings</u>

Figure 1 shows the Acquisition test stand set up.
Figure 2 shows the Post Acquisition Collagen Rewet Method test set up.

<u>Detailed description</u>

As used herein, the term "absorbent articles" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored as an absorbent article after being soiled or loaded (i.e., they are intended to be discarded after a single loading or soiling and, preferably, to be recycled, composted or otherwise disposed of in an environmentally compatible manner). Within the context of the present invention, the disposable absorbent article comprises two elements, one of which - namely the inner one positioned towards the body of the wearer - is intended to be disposed upon each "change" by a fresh one, whereas the other - namely the outer one, i.e. the one which is positioned on the garment side - can be used over several wearing periods of the inner one. Thus the outer element is still "disposable" for "one time usage", but this usage period can extend over one or more usage periods of the inner element. When looking at the total usage period of the outer element, there can be several inner elements used in combination therewith, however, for the following the term "an absorbent article" refers to a combination of one inner and one outer element as being worn together at one point in time.

The intended user group for articles according to the present invention are incontinent adults, but also babies and the like, i.e., generally users providing significant urine loads for the article, and - at least for part of the changes - also faecal material loading.

The articles according to the present invention are generally worn on or about the lower torso of the wearer. The intended use is such that both the primary outer and the secondary inner element are applied to the user. These elements are arranged such that the loading will be first caught by the secondary (inner) element. If desired, this can then be changed and be replaced by a fresh secondary (inner) element. If not changed sufficiently quickly after an insult, or if loaded with higher amounts of loading, such as with faeces, it can happen, that also the primary element will be loaded and/or soiled. Thus this primary (outer) element can be used over one or more usage cycles for the secondary (inner) element.

The primary and secondary elements of the present invention comprise both chassis and core components, i.e. both elements provide absorbency, and both elements provide means for sealing against the outside, namely a backsheet, and both can comprise further means for fixation and the like.

Primary absorbent element

The primary absorbent element generally comprises

- a primary absorbent core (which may consist of sub-structures);
- a fluid pervious topsheet;
- a fluid impervious backsheet;
- optionally further features like closure elements or elastification.

The primary element comprises a liquid pervious topsheet on the wearer oriented surface of the primary element; a liquid impervious backsheet on the opposite surface of the primary element facing away from the wearer; an absorbent core positioned between the topsheet and the backsheet.

Often the topsheet and the backsheet have dimensions larger than the absorbent core so as to allow combination of the two in the peripheral regions surrounding the core, thus providing structural integrity of the subelements.

While the topsheet, the backsheet, and the absorbent core may be assembled in a variety of well known configurations, preferred diaper configurations are described generally in U.S. Patent 3,860,003 entitled "Contractable Side Portions for Disposable Diaper" which issued to Kenneth B. Buell on January 14, 1975; and U.S. Patent Application Serial No. 07/715,152, allowed, "Absorbent Article With Dynamic Elastic Waist Feature Having A Predisposed Resilient Flexural Hinge", Kenneth B. Buell et al. filed June 13, 1991.

The backsheet may be secured to the absorbent core by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet is impervious to liquids (e.g. urine) and can be manufactured from a thin plastic film, although other flexible liquid impervious materials may also be used. As used herein, the term "flexible" refers to materials which are compliant and will readily conform to the general shape and contours of the human body. The backsheet may thus comprise a woven or nonwoven material, polymeric films such as thermoplastic films of polyethylene or polypropylene, or composite materials such as a film-coated nonwoven material. Thus, the backsheet can be a thermoplastic film having a thickness of from about 0.012 mm to about 0.051 mm. Such materials for the backsheet include RR8220 blown films and RR5475 cast films as manufactured by Tredegar Industries, Inc. of Terre Haute, IN, US. The backsheet can be embossed and/or matte finished to provide a more clothlike appearance. Preferably, the backsheet 26 may permit vapours to penetrate through (i.e. be breathable) while still preventing exudates from passing through the backsheet.

In a preferred embodiment, the primary element comprises an elasticised leg cuff, even more preferred additionally an elastic gasketing cuff, each with one or more elastic strands such as described in US Patent 4,695,278.

The primary element may further comprise an elastic waist feature that provides improved fit and containment, and preferably has two elastic waist features, one positioned in the front waist region and one positioned in the rear waist region. The primary element may further comprise other elastic features, such as elastic side panel, and allowing improved sustained fit of the article.

Preferably, the primary element comprises fixations means so as to keep the primary article in the appropriate positioning on the wearer. Thereby, the secondary element will be implicitly fixed, too, such that these means should be designed and dimensioned considering the secondary element. Such fixations means can be conventional adhesive tapes, or mechanical closure systems, but also can comprise topical adhesives to attaching parts of the primary element to the skin of the wearer.

Generally, such elastification or closure features are well-known in the art and - for example - described in EP 0254476 (Alemany) or WO 93/16669.

The primary element has as an essential feature a primary absorbent core element. As this is not intended to receive the most of the discharges and also not intended to be - in particular in the loading regions - in direct contact with the skin of the wearer, it has to satisfy certain but not very stringent absorbency requirements. Thereby, it has to have a sufficiently high liquid absorption capacity. It has been found that an ultimate storage capacity of 75 ml can be adequate for certain application, but that 90 ml are generally more preferred and 165 ml or more are most preferred.

Also, by its generally compressibility and conformability, the primary absorbent core element will contribute to the overall softness of the total article

Secondary absorbent element

The secondary element is intended to be positioned during use such that it is placed inside the primary (outer) element. Such an arrangement is well known from inserts, these, however, not satisfying the requirement of the present invention as to the specific fluid handling as laid out further. This secondary element comprises topsheet and the backsheet, essentially satisfying the same or similar requirement as for the primary element, also have length and width dimensions generally larger than those of the secondary absorbent core.

Optionally and preferably, the secondary element comprises fixation and sealing features like closure elements or elastification, which, however, do not need to allow support of both of the elements, but rather aim at supplementing the fixation and sealing elements of the primary element, e.g., to maintain and sustain fit of the article. Such fixation means can be adhesive applications such as well known as "panty fastening adhesive", whereby in the context of the present invention the fixation would not be with the underwear of the wearer, but rather with the primary element. Such adhesives could be uncovered immediately before use, or otherwise activated. Such adhesives could also be replaced by mechanical fixations means such as by well known hook-loop system, or simply by hook members attached to the backsheet of the secondary element for engagement with the surface, e.g. a nonwoven surface, of the primary element. Such fixation means can also be topical adhesives so as to fix the secondary element to the skin of the wearer.

In addition to these optional though partly preferred features, it is essential that the secondary absorbent element satisfies stringent liquid handling performance requirements, which can be achieved by the following materials and designs.

Secondary absorbent core element

The secondary absorbent core should be generally compressible, conformable, non-irritating to the wearer's skin, and capable of absorbing and retaining liquids such as urine and other certain body exudates. The secondary absorbent core has a garment surface ("lower" or "bottom" part), a body surface, side edges, and waist edges. The absorbent core might comprise a wide variety of liquid-absorbent or liquid handling materials commonly used in disposable diapers and other absorbent articles such as - but not limited to - comminuted wood pulp which is generally referred to as airfelt; meltblown polymers including coform; chemically stiffened, modified or cross-linked cellulosic fibres; tissue including tissue wraps and tissue laminates.

Examples for absorbent structures are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; EP-A-0 640 330 of Bewick-Sonntag et al.; US 5 180 622 (Berg et al.); US 5 102 597 (Roe et al.); US 5 387 207 (LaVon). Such structures might be adopted to be compatible with the requirements outline below for being used as the absorbent core 28.

The secondary absorbent core can be a unitary core structure, or it can be a combination of several absorbent sub-structures, which in turn can consist of further sub-structures. Each of the structure or sub-structures can have an essentially two-dimensional extension (i.e. be a layer) or a three-dimensional shape.

Materials for being used in secondary absorbent core element

The secondary absorbent core element for the present invention can comprise fibrous materials to form fibrous web or fibrous matrices.

Fibres useful in the present invention include those that are naturally occurring fibres (modified or unmodified), as well as synthetically made fibres, such as polyolefins as polyethylene and polypropylene.

Suitable naturally occurring fibres are wood pulp fibres which can be obtained from well-known chemical processes such as the Kraft and sulfite processes. Also chemically stiffened cellulosic fibres are suitable, wherein for example, crosslinking agents can be applied to the fibres that, subsequent to application, thus causing to chemically form intrafibre crosslink bonds which can increase the stiffness of the fibres. While the utilisation of intrafibre crosslink bonds to chemically stiffen the fibre is preferred, it is not meant to exclude other types of reactions for chemical stiffening of the fibres.

Fibres stiffened by crosslink bonds in individualised form (i.e., the individualised stiffened fibres, as well as process for their preparation) are disclosed, for example, in US-A-3,224,926; US-A-3,440,135; US-A-3,932,209; and US-A-4,035,147; US-A-4,898,642d; and US-A-5,137,537.

In addition to or alternatively synthetic or thermoplastic fibres can be comprised in the secondary absorbent structures, such as being made from any thermoplastic polymer that can be molten at temperatures that will not extensively damage the fibres. The thermoplastic materials, can be made from a variety of thermoplastic polymers, such as polyolefins such as polyethylene. The surface of the hydrophobic thermoplastic fibre can be rendered hydrophilic by treatment with a surfactant, such as a nonionic or anionic surfactant, e.g., by spraying the fibre with a surfactant, by dipping the fibre into a surfactant or by including the surfactant as part of the polymer melt in producing the thermoplastic fibre. Upon melting and re-solidification, the surfactant will tend to remain at the surfaces of the thermoplastic fibre.

Suitable thermoplastic fibres can be made from a single polymer (mono-component fibres), or can be made from more than one polymer (e.g., bi-component fibres). For example, "bi-component fibres" can refer to thermoplastic fibres that comprise a core fibre made from one polymer that is encased within a thermoplastic sheath made from a different polymer. The polymer comprising the sheath often melts at a different, typically lower, temperature than the polymer comprising the core. As a result, these bi-component fibres provide thermal bonding due to melting of the sheath polymer, while retaining the desirable strength characteristics of the core polymer.

In the case of thermoplastic fibres, their length can vary depending upon the particular melt point and other properties desired for these fibres. Typically, these thermoplastic fibres have a length from about 0.3 to about 7.5 cm long, preferably from about 0.4 to about 3.0 cm long. The properties, including melt point, of these thermoplastic fibres can also be adjusted by varying the diameter (caliper) of the fibres. The diameter of these thermoplastic fibres is typically defined in terms of either denier (grams per 9000 meters) or decitex (grams per 10,000 meters dtex). Depending on the specific arrangement within the structure, suitable thermoplastic fibres can have a decitex in the range from well below 1 decitex, such as 0.4 decitex to about 20 dtex.

Said fibrous materials may be used in an individualised form when the absorbent article is being produced, and an airlaid fibrous structure is formed on the line. Said fibres may also be used as a preformed fibrous web or tissue. These structures are then delivered to the production of the article essentially in endless or very long form (e.g. on a roll, spool) and will then be cut to the appropriate size. This can be done on each of such materials individually before these are combined with other materials to form the absorbent core, of when the core itself is cut and said materials are co-extensive with the core. There is a wide variety of making such webs or tissues, and such processes are very well known in the art.

In addition or alternatively to fibrous webs, the absorbent cores may comprise other porous materials, such as foams. Preferred foams are open-celled absorbent polymeric foam materials as being derived by polymerising a High Internal Phase Water-in-Oil Emulsion (hereafter referred to a HIPE). Such polymeric foams may be formed to provide the requisite storage properties, as well as the requisite distribution properties, such as described in U.S. Patent 5,650,222 (Application Serial No. 08/563,866; DesMarais et al.), filed November 25, 1995 (hereafter referred to as "'866 application"), copending U.S. Patent 5,849,805 (Application Serial No. 08/542,497, filed October 13, 1995; Dyer et al.); U.S. Patent 5,387,207 (Dyer et al.), issued February 7, 1995; and U.S. Patent 5,260,345 (DesMarais et al.), issued November 9, 1993;

Superabsorbent polymers or hydrogels

Optionally, and often preferably, the absorbent structures according to the present invention can comprise Superabsorbent polymers, or hydrogels. The hydrogel-forming absorbent polymers useful in the present invention include a variety of substantially water-insoluble, but water-swellable polymers capable of absorbing large quantities of liquids. Such polymer materials are also commonly referred to as "hydrocolloids", or "superabsorbent" materials. These hydrogel-forming absorbent polymers preferably have a multiplicity of anionic, functional groups, such as sulfonic acid, and more typically carboxy, groups. Examples of polymers suitable for use herein include those which are prepared from polymerisable, unsaturated, acid-containing monomers.

Hydrogel-forming absorbent polymers suitable for the present invention contain carboxy groups. These polymers include hydrolysed starch-acrylonitrile graft copolymers, partially neutralised starch-acrylonitrile graft copolymers, starch-acrylic acid graft copolymers, partially neutralised starch-acrylic acid graft copolymers, saponified vinyl acetate-acrylic ester copolymers, hydrolysed acrylonitrile or acrylamide copolymers, slightly network crosslinked polymers of any of the foregoing copolymers, partially neutralised polyacrylic acid, and slightly network crosslinked polymers of partially neutralised polyacrylic acid. These polymers can be used either solely or in the form of a mixture of two or more different polymers. Examples of these polymer materials are disclosed in U.S. Patent 3,661,875, U.S. Patent 4,076,663, U.S. Patent 4,093,776, U.S. Patent 4,666,983, and U.S. Patent 4,734,478.

Most preferred polymer materials for use in making hydrogel-forming particles are slightly network crosslinked polymers of partially neutralised polyacrylic acids and starch derivatives thereof. Most preferably, the hydrogel-forming particles comprise from about 50 to about 95%, preferably about 75%, neutralised, slightly network crosslinked, polyacrylic acid (i.e. poly (sodium acrylate/acrylic acid)).

As described above, the hydrogel-forming absorbent polymers are preferably slightly network crosslinked. Network crosslinking serves to render the polymer substantially water-insoluble and, in part, determines the absorptive capacity and extractable polymer content characteristics of the precursor particles and the resultant macrostructures. Processes for network crosslinking the polymers and typical network crosslinking agents are described in greater detail in the herein before-referenced U.S. Patent 4,076,663, and in DE-A-4020780 (Dahmen).

The superabsorbent materials can be used in particulate form or in fibrous form and may also be combined other elements to form preformed structures.

Whilst the individual elements have been disclosed separately, an absorbent structure or substructure can be made by combining one or more of these elements.

Interaction of primary and secondary absorbent elements

An essential feature of the present invention is the interactive effect of the two elements, namely that the secondary element is first loaded during use. Thereby, the high performance of the secondary element, such as expressed in superior rewet performance such as in PACORM testing, ensures good skin conditions, such as skin dryness.

For certain loading situations, however, even the high performance of this inner element is not sufficient, and the primary element will also be loaded, either because of excessive liquid loads, or by faeces, and thus prevents leakage.

Henceforth, the functionality of the prior art articles is essentially inverted, in so far as according to the present invention the primary element supports the secondary element, whilst in the prior art constructions the secondary (insert) element supports the outer one.

In most instances, the secondary element will be changed upon loading, either by the wearer him/herself, or by caretakers like nurses or parents.

As in most instances the primary element is not soiled at all, it can be reused several times, either until an exceptional loading occurs, or after a prolonged wearing time, a fresh primary element can be used.

Thus, the present invention requires, that the total article has an ultimate storage capacity of at least 165 ml, preferably 240 ml, but in the more preferred executions at least 330 ml, and most preferably more than 375 ml. or even more than 390 ml. Further, it is required, that the secondary element has an ultimate storage capacity of more than 75 ml, preferably 90 ml, but in the more preferred executions at least 165 ml, and most preferably more than 300 ml.

Thus, the secondary element provides at least 55 %, preferably more than 65 % but even more preferably more than 75 % and most preferably more than 80 % of the total ultimate storage capacity of the absorbent article.

In addition, the secondary element has to satisfy low rewet requirements, such as expressed in low PACORM values, such as less than about 100 mg, even more preferably less than 80 mg, but most preferably 72 mg or less.

Test procedures

Acquisition Test

This test should be carried out at about 22 +/- 2°C and at 35+/- 15% relative humidity. The synthetic urine used in these test methods is commonly known as Jayco SynUrine and is available from Jayco

Pharmaceuticals Company of Camp Hill, Pennsylvania. The formula for the synthetic urine is: 2.0 g/l of KCl; 2.0 g/l of $Na_2SO_4$; 0.85 g/l of $(NH_4)H_2PO_4$; 0.15 g/l $(NH_4)H_2PO_4$; 0.19 g/l of $CaCl_2$; ad 0.23 g/l of $MgCl_2$. All of the chemicals are of reagent grade. The pH of the synthetic Urine is in the range of 6.0 to 6.4.

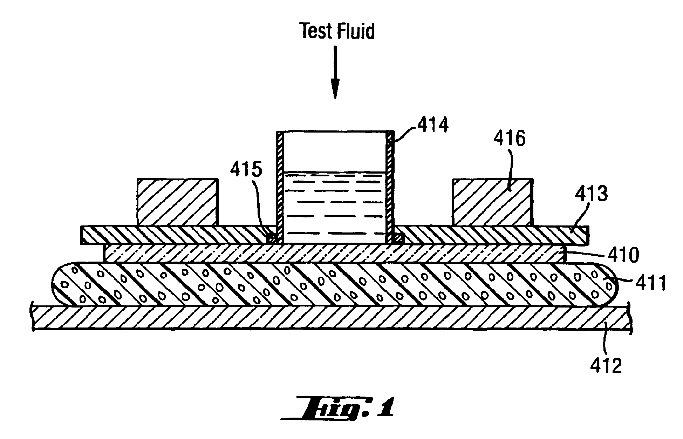

Referring to Figure 1, an absorbent structure (410) is loaded with a 75 ml gush of synthetic urine at a rate of 15 ml/s using a pump (Model 7520-00, supplied by Cole Parmer Instruments., Chicago, U.S.A.), from a height of 5 cm above the sample surface. The time to absorb the urine is recorded by a timer. The gush is repeated at precisely 5 minute gush intervals until the article is sufficiently loaded. Current test data are generated by loading four times.

The test sample, which can be a complete absorbent article or an absorbent structure comprising an absorbent core, a topsheet, and a backsheet, is arranged to lie flat on a foam platform 411 within a perspex box (only base 412 of which is shown). A perspex plate 413 having a 5 cm diameter opening in its middle is placed on top of the sample on the loading zone of the structure. Synthetic urine is introduced to the sample through a cylinder 414 fitted, and glued into the opening. Electrodes 415 are located on the lowest surface of the plate, in contact with the surface of the absorbent structure 410. The electrodes are connected to the timer. Loads 416 are placed on top of the plate to simulate, for example a baby's weight. A pressure of about 50g cm-2 (0.7psi) is achieved by positioning weights 416, e.g. for the commonly available MAXI size 20 kg.

As test fluid is introduced into the cylinder it typically builds up on top of the absorbent structure thereby completing an electrical circuit between the electrodes. The test fluid is transported from the pump to the test assembly by means of a tubing of about 8 mm diameter, which is kept filled with test fluid. Thus the fluid starts to leave the tubing essentially at the same time the pump starts operating. At this time, also the timer is started, and the timer is stopped when the absorbent structure has absorbed the gush of urine, and the electrical contact between the electrodes is broken.

The acquisition rate is defined as the gush volume absorbed (ml) per unit time(s). The acquisition rate is calculated for each gush introduced into the sample. Of particular interest in view of the current invention are the first and the last of the four gushes.

This test is primarily designed to evaluate products generally referred to as MAXI size products for a design capacity of about 300 ml, and having a respective Ultimate Storage Capacity of about 300 ml to 400 ml. If products with significantly different capacities should be evaluated (such as can be envisaged for adult incontinence products), the settings in particular of the fluid volume per gush should be adjusted appropriately to about 20% of the total article design capacity, and the deviation from the standard test protocol should be recorded.

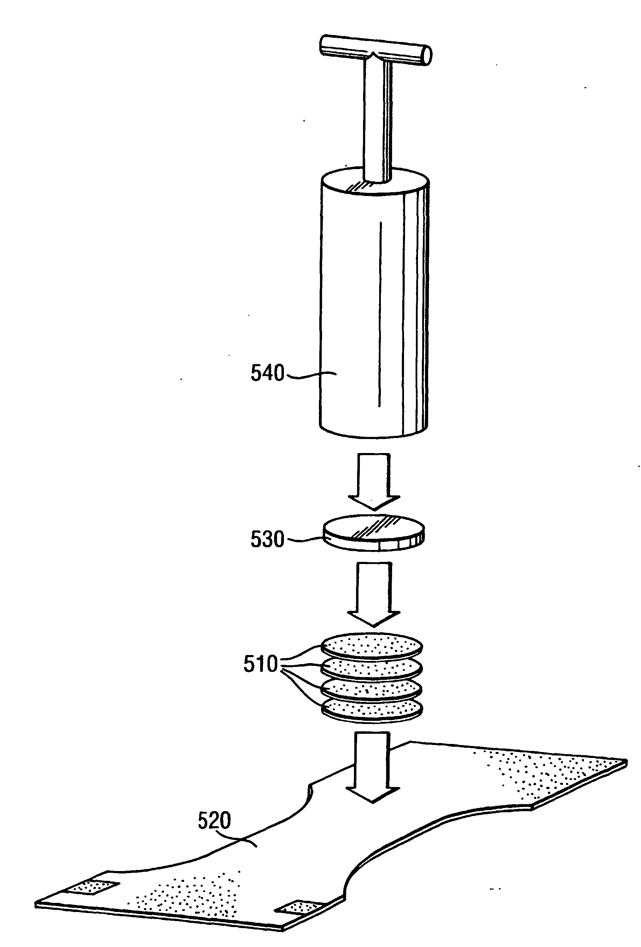

Post Acquisition Collagen Rewet Method (refer to Fig. 2)

Before executing the test, the collagen film as purchased from NATURIN GmbH, Weinhein, Germany, under the designation of COFFI and at a basis weight of about 28g/m$^2$ is prepared by being cut into sheets of 90 mm diameter e.g. by using a sample cutter device, and by equilibrating the film in the controlled environment of the test room (see above) for at least 12 hours (tweezers are to be used for all handling of the collagen film).

At least 5 minutes, but not more than 6 minutes after the last gush of the above acquisition test is absorbed, the cover plate and weights are removed, and the test sample (520) is carefully placed flat on a lab bench.

Four sheets of the precut and equilibrated collagen material (510) are weighed with at least one milligram accuracy, and then positioned centred onto the loading point of the article, and covered by perspex plate (530) of 90 mm diameter, and about 20 mm thickness. A weight (540) of 15 kg is carefully added (also centred). After 30 +/- 2 seconds the weight and perspex plate are carefully removed again, and the collagen films are reweighed.

The Post Acquisition Collagen Rewet Method result is the moisture pick up of the collagen film, expressed in mg.

It should be noted further, that this testing protocol can be adjusted easily according to specific product types, such as different baby diaper sizes, or adult incontinence articles, or catamenial articles, or by the variation in the type and amount of loading fluid, the amount and size of the absorbent material, or by variations in the applicable pressure. Having once defined these relevant parameters, such modifications will be obvious to one skilled in the art. When considering the results from the adjusted test protocol the products can easily be optimising these identified relevant parameters such as in a designed experiment according to standard statistical methods with realistic in use boundary conditions.

Teabag Centrifuge Capacity Test (TCC test)

Whilst the TCC test has been developed specifically for superabsorbent materials, it can readily be applied to other absorbent materials.

The Teabag Centrifuge Capacity test measures the Teabag Centrifuge Capacity values, which are a measure of the retention of liquids in the absorbent materials.

The absorbent material is placed within a "teabag", immersed in a 0.9% by weight sodium chloride solution for 20 minutes, and then centrifuged for 3 minutes. The ratio of the retained liquid weight to the initial weight of the dry material is the absorptive capacity of the absorbent material.

Two liters of 0.9% by weight sodium chloride in distilled water is poured into a tray having dimensions 24 cm x 30 cm x 5 cm. The liquid filling height should be about 3 cm.

The teabag pouch has dimensions 6.5 cm x 6.5 cm and is available from Teekanne in Düsseldorf, Germany. The pouch is heat sealable with a standard kitchen plastic bag sealing device (e.g. VACUPACK2 PLUS from Krups, Germany).

The teabag is opened by carefully cutting it partially, and is then weighed. About 0.200g of the sample of the absorbent material, accurately weighed to +/- 0.005g, is placed in the teabag. The teabag is then closed with a heat sealer. This is called the sample teabag. An empty teabag is sealed and used as a blank.

The sample teabag and the blank teabag are then laid on the surface of the saline solution, and submerged for about 5 seconds using a spatula to allow complete wetting (the teabags will float on the surface of the saline solution but are then completely wetted). The timer is started immediately. After 20 minutes soaking time the sample teabag and the blank teabag are removed from the saline solution, and placed in a Bauknecht WS130, Bosch 772 NZK096 or equivalent centrifuge (230 mm diameter), so that each bag sticks to the outer wall of the centrifuge basket. The centrifuge lid is closed, the centrifuge is started, and the speed increased quickly to 1,400 rpm. Once the centrifuge has been stabilised at 1,400 rpm the timer is started. After 3 minutes, the centrifuge is stopped.

The sample teabag and the blank teabag are removed and weighed separately.

The Teabag Centrifuge Capacity (TCC) for the sample of absorbent material is calculated as follows:

$$TCC = [(\text{sample teabag weight after centrifuging}) - (\text{blank teabag weight after centrifuging}) - (\text{dry absorbent material weight})] \div (\text{dry absorbent material weight}).$$

Also, specific parts of the structures or the total absorbent articles can be measured, such as "sectional" cut outs, i.e. looking at parts of the structure or the total article, whereby the cutting is done across the full width of the article at determined points of the longitudinal axis of the article. In particular, the definition of the "crotch region" as described above allows to determine the "crotch region capacity". Other cut-outs can be used to determine a "basis capacity" (i.e. the amount of capacity contained in a unit area of the specific region of the article). Depending on the size of the unit area (preferably 2 cm by 2 cm) these defines how much averaging is taking place - naturally, the smaller the size, the less averaging will occur.

Ultimate Storage Capacity

In order to determine or evaluate the Ultimate Design Storage Capacity of an absorbent article, a number of methods have been proposed.

In the context of the present invention, it is assumed, that the Ultimate Storage Capacity of an article is the sum of the ultimate absorbent capacities of the individual elements or material. For these individual components, various well established techniques can be applied as long as these are applied consistently throughout the comparison. For example, the Tea Bag Centrifuge Capacity as developed and well established for superabsorbent polymers (SAP) can be used for such SAP materials, but also for others (see above).

Once the capacities for the individual materials are known, the total article capacity can be calculated by multiplying these values (in ml/g) with the weight of the material used in the article.

For materials having a dedicated functionality other than ultimate storage of fluids - such as acquisition layers and the like - the ultimate storage capacity can be neglected, either as such materials do in fact have only very low capacity values compared to the dedicated ultimate fluid storage materials, or as such materials are intended to not be loaded with fluid, and thus should release their fluid to the other ultimate storage materials.

**Claims**

1. Absorbent article,
   having a total ultimate storage capacity of at least 240 ml. comprising
   an outer absorbent element comprising
   a primary element comprising primary chassis and primary absorbent core elements;
   and an inner element positioned towards the wearer comprising a secondary element comprising secondary chassis and secondary absorbent core elements;
   **characterised in that**
   the secondary absorbent core element has a secondary ultimate storage capacity of more than 75 % of the total article ultimate storage capacity and of at least 165 ml,
   and **in that** the secondary absorbent core element provides a rewet of less than 100 mg according to the Post Acquisition Collagen Rewet Method described herein.

**2.** Absorbent article, according to claim 1,
further **characterised in that**
the article has a total ultimate storage capacity of more than 330 ml.

**3.** Absorbent article, according to claim 1,
further **characterised in that**
the article has a total ultimate storage capacity of more than 375 ml.

**4.** Absorbent article, according to claim 1,
further **characterised in that**
the article has a total ultimate storage capacity of more than 390 ml.

**5.** Absorbent article, according to claim 1,
further **characterised in that**
the secondary element has an ultimate storage capacity of more than 80 % of the total ultimate storage capacity.

**6.** Absorbent article, according to any of the preceding claims,
further **characterised in that**
the secondary element has a rewet of less than 80 mg according to the Post Acquisition Collagen Rewet Method described herein.

**7.** Absorbent article, according to claim 10,
further **characterised in that**
the secondary element has a rewet of less than 70 mg according to the Post Acquisition Collagen Rewet Method described herein.

**8.** Absorbent article, according to any of the preceding claims, wherein the absorbent article is an adult incontinence article.

**9.** Absorbent article, according to any of the preceding claims, wherein the absorbent article is a baby diaper.

**Patentansprüche**

**1.** Absorptionsartikel,
der eine elementare gesamte Speicherkapazität von mindestens 240 ml aufweist, umfassend
ein äußeres absorbierendes Element, das ein primäres Element, umfassend primäre Grundeinheits- und primäre Absorptionskernelemente, umfasst;
und ein inneres Element, das zum Träger gerichtet positioniert ist und das ein sekundäres Element, umfassend sekundäre Grundeinheits- und sekundäre Absorptionskemelemente, umfasst;
**dadurch gekennzeichnet, dass**
das sekundäre Absorptionskernelement eine elementare sekundäre Speicherkapazität von mehr als 75 % der gesamten elementaren Speicherkapazität des Artikels und von mindestens 165 ml aufweist,
und **dadurch**, dass das sekundäre Absorptionskernelement eine Rücknässung von weniger als 100 mg gemäß dem hierin erläuterten Verfahren der Collagenwiederbenetzung nach Aufnahme aufweist.

**2.** Absorptionsartikel nach Anspruch 1,
ferner **dadurch gekennzeichnet, dass**
der Artikel eine gesamte elementare Speicherkapazität von mehr als 330 ml aufweist.

**3.** Absorptionsartikel nach Anspruch 1,
ferner **dadurch gekennzeichnet, dass**
der Artikel eine gesamte elementare Speicherkapazität von mehr als 375 ml aufweist.

**4.** Absorptionsartikel nach Anspruch 1,
ferner **dadurch gekennzeichnet, dass**
der Artikel eine gesamte elementare Speicherkapazität von mehr als 390 ml aufweist.

**5.** Absorptionsartikel nach Anspruch 1,
ferner **dadurch gekennzeichnet, dass**
das sekundäre Element eine elementare Speicherkapazität von mehr als 80 % der gesamten elementaren Speicherkapazität aufweist.

**6.** Absorptionsartikel nach einem der vorstehenden Ansprüche,
ferner **dadurch gekennzeichnet, dass**
das sekundäre Element eine Rücknässung von weniger als 80 mg gemäß dem hierin erläuterten Verfahren der Collagenwiederbenetzung nach Aufnahme aufweist.

**7.** Absorptionsartikel nach Anspruch 10,
ferner **dadurch gekennzeichnet, dass**
das sekundäre Element eine Rücknässung von weniger als 70 mg gemäß dem hierin erläuterten Verfahren der Collagenwiederbenetzung nach Aufnahme aufweist.

**8.** Absorptionsartikel nach einem der vorstehenden Ansprüche,
wobei der Absorptionsartikel ein Inkontinenzartikel für Erwachsene ist.

**9.** Absorptionsartikel nach einem der vorstehenden Ansprüche,
wobei der Absorptionsartikel eine Babywindel ist.

**Revendications**

**1.** Article absorbant,
ayant une capacité de stockage ultime totale d'au moins 240 ml
comprenant
un élément absorbant externe comprenant un élément primaire comprenant des éléments de châssis primaire et d'âme absorbante primaire;
et un élément interne positionné vers le porteur comprenant un élément secondaire comprenant des éléments de châssis secondaire et d'âme absorbante secondaire;
**caractérisé en ce que**
l'élément d'âme absorbante secondaire a une capacité de stockage ultime secondaire de plus de 75 % de la capacité de stockage ultime totale et d'au moins 165 ml,
et **en ce que** l'élément d'âme absorbante secondaire fournit un remouillage de moins de 100 mg selon le procédé de remouillage au collagène après recueil traité ici.

**2.** Article absorbant selon la revendication 1,
**caractérisé, en outre, en ce que**
l'article a une capacité de stockage ultime totale de plus de 330 ml.

**3.** Article absorbant selon la revendication 1,
**caractérisé, en outre, en ce que**
l'article a une capacité de stockage ultime totale de plus de 375 ml.

**4.** Article absorbant selon la revendication 1,
**caractérisé, en outre, en ce que**
l'article a une capacité de stockage ultime totale de plus de 390 ml.

**5.** Article absorbant selon la revendication 1,
**caractérisé, en outre, en ce que**
l'élément secondaire a une capacité de stockage ultime de plus de 80 % de la capacité de stockage ultime totale.

**6.** Article absorbant selon l'une quelconque des revendications précédentes,
**caractérisé, en outre, en ce que**
l'élément secondaire a un remouillage de moins de 80 mg selon le procédé de remouillage au collagène après recueil traité ici.

**7.** Article absorbant selon la revendication 10,
**caractérisé, en outre, en ce que**
l'élément secondaire a un remouillage de moins de 70 mg selon le procédé de remouillage au collagène après recueil traité ici.

**8.** Article absorbant selon l'une quelconque des revendications précédentes,
où l'article absorbant est un article pour l'incontinence de l'adulte.

**9.** Article absorbant selon l'une quelconque des revendications précédentes,
où l'article absorbant est une couche pour bébés.

Test Fluid

414

416

415

413

410

411

412

*Fig. 1*